(19) **Europäisches Patentamt / European Patent Office / Office européen des brevets**

(11) **EP 4 111 976 B1**

(12) **FASCICULE DE BREVET EUROPEEN**

(45) Date de publication et mention de la délivrance du brevet:
**31.07.2024 Bulletin 2024/31**

(21) Numéro de dépôt: **22182367.7**

(22) Date de dépôt: **30.06.2022**

(51) Classification Internationale des Brevets (IPC):
**A61B 5/369** (2021.01)    **G06F 3/01** (2006.01)

(52) Classification Coopérative des Brevets (CPC):
**A61B 5/369; G06F 3/015; A61B 2560/0223**

(54) **MÉTHODE DE CALIBRATION EN LIGNE D'UNE INTERFACE NEURONALE DIRECTE AVEC DÉTERMINATION DES HYPERPARAMÈTRES AU MOYEN D'UN APPRENTISSAGE PAR RENFORCEMENT**

ONLINE-KALIBRIERUNGSMETHODE FÜR EINE GEHIRN-COMPUTER-SCHNITTSTELLE MIT BESTIMMUNG VON HYPERPARAMETERN DURCH VERSTÄRKUNGSLERNEN

ONLINE CALIBRATION METHOD FOR A BRAIN COMPUTER INTERFACE WITH DETERMINATION OF HYPERPARAMETERS BY REINFORCEMENT LEARNING

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **30.06.2021 FR 2107070**

(43) Date de publication de la demande:
**04.01.2023 Bulletin 2023/01**

(73) Titulaire: **Commissariat à l'énergie atomique et aux énergies alternatives**
**75015 Paris (FR)**

(72) Inventeurs:
- **MOLY, Alexandre**
  **38054 GRENOBLE CEDEX 09 (FR)**
- **AKSENOVA, Tetiana**
  **38054 GRENOBLE CEDEX 09 (FR)**

(74) Mandataire: **Brevalex**
**56, Boulevard de l'Embouchure**
**B.P. 27519**
**31075 Toulouse Cedex 2 (FR)**

(56) Documents cités:
**EP-A1- 3 190 480    US-A1- 2021 064 942**

- **MOLY ALEXANDRE: "Innovative decoding algorithms for Chronic ECoG-based Brain Computer Interface (BCI) for motor disabled subjects in laboratory and at home", 10 December 2020 (2020-12-10), XP055869052, Retrieved from the Internet <URL:https://thares.univ-grenoble-alpes.fr/2020 GRALS028.pdf> [retrieved on 20211203]**
- **ALEXANDRE MOLY ET AL: "Online adaptive group-wise sparse NPLS for ECoG neural signal decoding", ARXIV.ORG, CORNELL UNIVERSITY LIBRARY, 201 OLIN LIBRARY CORNELL UNIVERSITY ITHACA, NY 14853, 14 October 2020 (2020-10-14), XP081791002**
- **ANDREY ELISEYEV ET AL: "Recursive Exponentially Weighted N-way Partial Least Squares Regression with Recursive-Validation of Hyper-Parameters in Brain-Computer Interface Applications", NATURE, SCIENTIFIC REPORTS, vol. 7, no. 1, 24 November 2017 (2017-11-24), XP055705682, DOI: 10.1038/s41598-017-16579-9**

**Description**

**DOMAINE TECHNIQUE**

L'objet de la présente invention concerne le domaine des interfaces neuronales directes encore dénommées BCI (*Brain Computer Interface*) ou BMI (*Brain Machine Interface*). Elle trouve notamment à s'appliquer à la commande neuronale directe d'une machine, telle qu'un exosquelette ou un ordinateur.

**ÉTAT DE LA TECHNIQUE ANTÉRIEURE**

Les interfaces neuronales directes utilisent les signaux électro-physiologiques émis par le cortex cérébral pour élaborer un signal de commande. Ces interfaces neuronales ont fait l'objet de nombreuses recherches notamment dans le but de restaurer une fonction motrice à un sujet paraplégique ou tétraplégique à l'aide d'une prothèse ou d'une orthèse motorisée.

Les interfaces neuronales peuvent être de nature invasive ou non invasive. Les interfaces neuronales invasives utilisent des électrodes intracorticales (c'est-à-dire implantées dans le cortex) ou des électrodes corticales (disposées à la surface du cortex) recueillant dans ce dernier cas des signaux électrocorticographiques (EcoG). Les interfaces neuronales non invasives utilisent des électrodes placées sur le cuir chevelu pour recueillir des signaux électroencéphalographiques (EEG). D'autres types de capteurs ont été également envisagés comme des capteurs magnétiques mesurant les champs magnétiques induits par l'activité électrique des neurones du cerveau. On parle alors de signaux magnétoencéphalographiques (MEG).

Quel que soit le type des signaux recueillis (EEG, ECoG, MEG), l'interface BCI doit effectuer un traitement de signal afin d'estimer la trajectoire du mouvement désiré par le sujet et en déduire les signaux de commande de l'ordinateur ou de la machine. Par exemple, lorsqu'il s'agit de commander un exosquelette, l'interface BCI estime la trajectoire du mouvement désiré à partir des signaux électro-physiologiques mesurés et en déduit les signaux de contrôle permettant à l'exosquelette de reproduire la trajectoire en question. De manière similaire, lorsqu'il s'agit de commander un ordinateur, l'interface BCI estime par exemple la trajectoire souhaitée d'un pointeur ou d'un curseur à partir des signaux électrophysiologiques et en déduit les signaux de commande du curseur/pointeur.

L'estimation de trajectoire, encore dénommée décodage neuronal dans la littérature, nécessite généralement une phase d'apprentissage ou de calibration préalable. Lors de cette phase, le sujet imagine, observe ou réalise un mouvement selon une trajectoire déterminée pendant un intervalle de calibration donné. Les signaux électro-physiologiques mesurés pendant cet intervalle sont exploités en relation avec la trajectoire déterminée pour construire un modèle prédictif, et plus précisément pour calculer les paramètres de ce modèle. En pratique, la phase de calibration prend en compte un grand nombre de mouvements successifs, les paramètres du modèle prédictif étant corrigés en fonction des erreurs de prédiction.

L'interface BCI utilise ensuite le modèle prédictif ainsi obtenu pour estimer la trajectoire souhaitée à partir des signaux électro-physiologiques mesurés.

De nombreuses méthodes de construction de modèle prédictif pour interface neuronale directe ont été proposées dans l'état de la technique. La méthode de régression des moindres carrés partiels ou PLS (*Partial Least Squares regression*) fait partie des plus prometteuses en raison de sa capacité à réduire la dimension des vecteurs d'observation. On rappelle que la méthode PLS est une méthode permettant de déterminer un modèle de prédiction linéaire entre des variables d'entrée (ou variables prédictives) et des variables de sortie en faisant appel à des variables latentes (c'est-à-dire inobservables) explicatives des variables de sortie et des variables d'entrée, et liées par une régression linéaire.

On trouvera une description détaillée de la méthode PLS dans l'article de P. Geladi et al. intitulé « Partial least-squares régression : a tutorial » publié dans Analytica Chimica Acta, 185, pages 1-17, 1986, ainsi que dans la thèse de S. Vancolen intitulée « La régression PLS », Juin 2004.

Plus précisément, on note $\mathbf{x}_k$ le vecteur (de dimension $n$, où $n$ est le nombre d'électrodes) des signaux neuronaux acquis à l'instant $t_k$, et $\mathbf{y}_k$ le vecteur (de dimension $m$) des variables décrivant la trajectoire (par exemple $m = 9$, les variables étant les coordonnées dans l'espace du point de la trajectoire, les composantes du vecteur vitesse et les composantes du vecteur accélération à l'instant considéré). On forme la matrice d'observations $\mathbf{X} = (\mathbf{x}_1,...,\mathbf{x}_N)^T$ de taille $N \times n$ représentant les signaux neuronaux en $N$ instants successifs et la matrice d'observations $\mathbf{Y} = (\mathbf{y}_1,...,\mathbf{y}_N)^T$ detaille $N \times m$ décrivant la trajectoire en ces mêmes instants. L'algorithme PLS est un processus itératif permettant de modéliser une relation linéaire entre des variables d'entrée et des variables de sortie sur la base de leurs observations respectives (ici la matrice d'observation $\mathbf{X}$ pour les variables d'entrée et la matrice d'observation $\mathbf{Y}$ pour les variables de sortie), soit :

$$\mathbf{Y} = \mathbf{XB} + \mathbf{V} \tag{1}$$

où **B** est une matrice de coefficients et **V** une matrice de bruit. Les matrices **X** et **Y** sont projetées dans des espaces de faibles dimensions (espace des variables latentes) de manière à expliquer simultanément les variations de **X** et **Y** .

Lors de la première itération, la projection sur les espaces des variables latentes peut s'exprimer par :

$$\mathbf{X} = \mathbf{t}_1 \mathbf{p}_1^{\mathbf{T}} + \mathbf{E}_1 \qquad (2\text{-}1)$$

$$\mathbf{Y} = \mathbf{u}_1 \mathbf{q}_1^{\mathbf{T}} + \mathbf{F}_1 \qquad (2\text{-}2)$$

où $\mathbf{t}_1, \mathbf{u}_1$ sont des vecteurs de variables latentes (encore dénommés vecteurs de cotes ou « *score vectors* »), $\mathbf{p}_1, \mathbf{q}_1$ sont des vecteurs de charges ou « *looding vectors* ») et $\mathbf{E}_1, \mathbf{F}_1$ sont des matrices résiduelles. Les vecteurs de cotes sont calculés de manière à maximiser la covariance entre $\mathbf{t}_1$ et $\mathbf{u}_1$ et le coefficient de régression $b_1$ entre $\mathbf{t}_1$ et $\mathbf{u}_1$ est déterminé par la méthode des moindres carrés :

$$\mathbf{u}_1 = b_1 \mathbf{t}_1 + \mathbf{r}_1 \qquad (2\text{-}3)$$

en minimisant le vecteur résiduel $\mathbf{r}_1$.

A la seconde itération, on applique le même processus aux matrices résiduelles, $\mathbf{E}_1, \mathbf{F}_1$. De manière générale à l'itération $f + 1$, les matrices considérées sont :

$$\mathbf{X}_{f+1} = \mathbf{X}_f - \mathbf{t}_f \mathbf{p}_f^T \qquad (3\text{-}1)$$

$$\mathbf{Y}_{f+1} = \mathbf{Y}_f - \mathbf{u}_f \mathbf{q}_f^T \qquad (3\text{-}2)$$

On obtient en définitive, de proche en proche, au bout de $F$ itérations :

$$\mathbf{X} = \mathbf{T}\mathbf{P}^{\mathbf{T}} + \mathbf{E} \qquad (4\text{-}1)$$

$$\mathbf{Y} = \mathbf{U}\mathbf{Q}^{\mathbf{T}} + \mathbf{F} \qquad (4\text{-}2)$$

où **T** = $(\mathbf{t}_1,...,\mathbf{t}_F)$ et **U** = $(\mathbf{u}_1,...,\mathbf{u}_F)$ sont respectivement les matrices des cotes, **P** = $(\mathbf{p}_1,...,\mathbf{p}_F)$ et **Q** = $(\mathbf{q}_1,....\mathbf{q}_F)$ sont les matrices des charges (encore dénommées matrices de pondération), et **E,F** sont des matrices résiduelles.

La méthode PLS a été généralisée en utilisant des tenseurs à la place de matrices pour décrire les variables d'entrée et de sortie du modèle. On rappelle qu'un vecteur et une matrice sont des cas particuliers de tenseurs respectivement d'ordre 1 et 2. La variable d'entrée est notée $\underline{\mathbf{X}} \in \mathbb{R}^{I_1 \times ... \times I_n}$ où $n$ est l'ordre du tenseur et $I_i$ est la dimension du mode $i$. Les modes peuvent être par exemple le temps (nombre d'échantillons dans le temps, la fréquence (nombre de bandes spectrales d'analyse), l'espace (nombre d'électrodes). La méthode PLS ainsi généralisée est dénommée méthode PLS multivariée ou NPLS (*N-way PLS*). On en trouvera une description détaillée dans la thèse de R. Bro intitulée « Multi-way analysis in the food industry : models, algorithms and applications », 1998. La méthode PLS multivariée a été appliquée au calcul du modèle prédictif des trajectoires dans une interface BCI comme décrit dans l'article de A. Eliseyev et T. Aksenova intitulé « Recursive N-Way Partial Least Squares for Brain-Computer Interface » publié dans PLOS One, Juillet 2013, vol. 8, n° 7, e69962 et dans la demande internationale WO-A-2013/057544. Selon cette méthode, le modèle prédictif est construit de manière itérative, en projetant les tenseurs (en lieu et place des matrices) sur les espaces de variables latentes, soit en transposant les expressions (4-1) et (4-2) :

$$\underline{\mathbf{X}} = \sum_{f=1}^{F} \mathbf{t}_f \circ \mathbf{w}_1^f \circ ... \circ \mathbf{w}_n^f + \underline{\mathbf{E}} \qquad (5\text{-}1)$$

$$\underline{\mathbf{Y}} = \sum_{f=1}^{F} \mathbf{u}_f \circ \mathbf{q}_1^f \circ \ldots \circ \mathbf{q}_n^f + \underline{\mathbf{F}} \qquad (5\text{-}2)$$

où $\circ$ est le produit tensoriel, et $\mathbf{t}_f$ et $\mathbf{u}_f$ sont liés par la relation de régression :

$$\mathbf{u}_f = \mathbf{T}_f \mathbf{B}_f \qquad (6)$$

où $\mathbf{T}_f = \left( \mathbf{t}_1, \ldots, \mathbf{t}_f \right) \in \mathbb{R}^{N \times f}$ et $\mathbf{U}_f = \left( \mathbf{u}_1, \ldots, \mathbf{u}_f \right) \in \mathbb{R}^{N \times f}$ sont respectivement les matrices des cotes à l'itération $f = 1, \ldots, F$, $\mathbf{B}_f$ est la matrice des coefficients de régression et où $\mathbf{w}_i^f \in \mathbb{R}^{I_i}$, $\mathbf{q}_j^f \in \mathbb{R}^{J_j}$ sont les vecteurs de pondération de norme égale à 1 et où $\underline{\mathbf{E}}, \underline{\mathbf{F}}$ sont des tenseurs résiduels.

Là encore on passe d'une itération à l'autre au moyen de :

$$\underline{\mathbf{X}}_{f+1} = \underline{\mathbf{X}}_f - \sum_{f=1}^{F} \mathbf{t}_f \circ \mathbf{w}_1^f \circ \ldots \circ \mathbf{w}_n^f \qquad (7\text{-}1)$$

$$\underline{\mathbf{Y}}_{f+1} = \underline{\mathbf{Y}}_f - \sum_{f=1}^{F} \mathbf{u}_f \circ \mathbf{q}_1^f \circ \ldots \circ \mathbf{q}_n^f \qquad (7\text{-}2)$$

L'étape de calibration fournit en définitive les vecteurs de pondération (encore appelés projecteurs) $\mathbf{w}_i^f \in \mathbb{R}^{I_i}$, $\mathbf{q}_j^f \in \mathbb{R}^{J_j}$, $i = 1..,n$, $j = 1,..,M$ et les matrices $\mathbf{B}_f$, $f = 1,..,F$. L'ensemble des matrices $\mathbf{B}_f$, $f = 1,..,F$ est appelé modèle de prédiction (ou modèle prédictif).

Lorsque l'interface BCI est ensuite en opération, la matrice $\mathbf{X}$ des signaux électro-physiologiques mesurés est projetée sur l'espace des variables latentes à l'aide des vecteurs de pondération $\mathbf{w}_i^f$ pour en déduire la matrice de cotes $\mathbf{T}_F$. On en déduit la matrice de cotes $\mathbf{U}_F$ au moyen de l'équation de régression, puis l'estimation $\hat{\underline{\mathbf{Y}}}$ de $\underline{\mathbf{Y}}$ à partir des vecteurs de pondération $\mathbf{q}_j^f$ (cf. expression 5-2).

De manière synthétique, l'estimation $\hat{\underline{\mathbf{Y}}}$ du tenseur de sortie (tenseur de commande d'un effecteur ou tenseur décrivant la trajectoire) à l'instant $t$ peut s'exprimer sous la forme tensorielle :

$$\hat{\underline{\mathbf{Y}}}_t = \underline{\mathbf{X}}_t \underline{\mathbf{B}} + \underline{\beta} \qquad (8)$$

où est le tenseur du modèle prédictif et $\underline{\beta}$ est un tenseur de biais.

La dimension optimale, $F^*$ de l'espace des variables latentes (ou de manière équivalente du nombre d'itérations successives dans la méthode PLS ou NPLS) est choisie de manière à minimiser l'erreur de prédiction entre l'estimation $\hat{\underline{\mathbf{Y}}}_t$ et l'observation $\underline{\mathbf{Y}}_t$ à l'instant courant à partir du modèle prédictif de l'instant précédent, $t$-1.

Lorsque la calibration de l'interface BCI est réalisée hors ligne (*offline*), la dimension optimale $F^*$ peut être obtenue au moyen d'une validation croisée entre un jeu de données d'entrainement et un jeu de données de test. En revanche, lorsque cette calibration est réalisée en ligne (*on line*), cette dimension optimale peut être obtenue au moyen d'un processus de validation récursif comme décrit dans l'article de A. Eliseyev et al. intitulé « Recursive exponentially weighted N-way partial least squares régression with recursive hyperparameters in Brain- Computer Interface applications", Sci. Rep., vol. 7, N° 1, p. 16281, nov. 2017.

La calibration ou, de manière équivalente, le mécanisme de mise à jour du modèle prédictif selon l'algorithme REW-NPLS (*Recursive Exponentially Weigthed N-way Partial Least Squares*) est rappelé ci-après.

Si l'on note $\{\underline{\mathbf{X}}_u, \underline{\mathbf{Y}}_u\}$ les blocs de données de calibration (*training data set*) lors de l'étape de calibration (encore dénommée étape d'apprentissage) $u$, l'idée de base de l'algorithme REW-NPLS est de mettre à jour le modèle prédictif à partir des tenseurs de covariance et de covariance croisée obtenus à l'étape de calibration précédente, respectivement notés $\underline{\mathbf{XX}}_{u-1}$ et $\underline{\mathbf{XY}}_{u-1}$. Ces deux tenseurs permettent d'obtenir une représentation condensée du modèle prédictif à l'étape précédente, sans qu'il soit besoin de stocker l'historique des blocs de données de calibration précédents : $\{\underline{\mathbf{X}}_1,\underline{\mathbf{Y}}_1\},\{\underline{\mathbf{X}}_2,\underline{\mathbf{Y}}_2\},...\{\underline{\mathbf{X}}_{u-1},\underline{\mathbf{Y}}_{u-1}\}$.

Plus précisément, les tenseurs de covariance et de covariance croisée sont mis à jour au moyen des relations de récurrence :

$$\underline{\mathbf{XX}}_u = \gamma \underline{\mathbf{XX}}_{u-1} + \underline{\mathbf{X}}_u \times_1 \underline{\mathbf{X}}_u \qquad (9\text{-}1)$$

$$\underline{\mathbf{XY}}_u = \gamma \underline{\mathbf{XY}}_{u-1} + \underline{\mathbf{X}}_u \times_1 \underline{\mathbf{Y}}_u \qquad (9\text{-}2)$$

où $\times_1$ désigne le produit de tenseur de mode 1 et $\gamma$ est un coefficient d'oubli avec $0<\gamma<1$.

On comprend ainsi qu'une période de calibration courante, réalisée lors de l'itération $u$ tient compte d'une période de calibration précédente réalisée lors de l'itération $u$-1 et des tenseurs des données d'observation de la période de calibration courante, l'historique étant pondéré par le coefficient d'oubli $\gamma$ avec $0 < \gamma < 1$.

Cette opération de mise à jour des tenseurs de covariance est effectuée de manière itérative sur les étapes de calibration.

Pour chaque mise à jour, le modèle prédictif est ensuite obtenu de manière itérative au moyen d'une décomposition PARAFAC (généralisation au cas tensoriel d'une décomposition en valeurs singulières) du tenseur de covariance croisée, $\underline{\mathbf{XY}}_u$. On rappelle que, d'une manière générale, une décomposition PARAFAC d'un tenseur $\underline{\mathbf{Z}}$ d'ordre $M$, soit

$$\underline{\mathbf{Z}} \in \mathbb{R}^{I_1 \times I_2 \times ... \times I_M}$$

permet de représenter ce tenseur sous la forme d'une combinaison linéaire de produits extérieurs de vecteurs (tenseurs d'ordre 1) à un tenseur résiduel près :

$$\underline{\mathbf{Z}} = \sum_{r=1}^{R} \theta_r \mathbf{w}_r^1 \circ \mathbf{w}_r^2 \circ ... \circ \mathbf{w}_r^M + \underline{\mathbf{E}} \qquad (10)$$

avec $\left\|\mathbf{w}_r^m\right\|=1$ , $m=1,..,M$ où $\circ$ est le produit extérieur et $\underline{\mathbf{E}}$ est un tenseur résiduel et $r$ est l'indice de l'itération PARAFAC et $R$ est un entier donné.

Dans le cas d'une étape de calibration REW-NPLS, chaque itération PARAFAC ne porte que sur une décomposition élémentaire, c'est-à-dire $R = 1$. Elle permet d'extraire du tenseur $\underline{\mathbf{XY}}_u$ un ensemble de vecteurs de projection, encore dénommés projecteurs, $\mathbf{w}_f^1,...,\mathbf{w}_f^M$ , de tailles respectives $I_1,I_2,..,I_M$ où le numéro d'itération $f$ donne la dimension d'un espace de variables latentes sur lequel est projeté le tenseur d'entrée $\underline{\mathbf{X}}_u$.

Afin de simplifier la présentation et sans préjudice de généralisation à un tenseur d'ordre quelconque, nous supposerons dans la suite que l'ordre du tenseur $\underline{\mathbf{XY}}_u$ est égal à 3 ($M = 3$), c'est-à-dire $\underline{\mathbf{XY}}_u \in \mathbb{R}^{I_1 \times I_2 \times I_3}$ avec $\|\underline{\mathbf{XY}}_u\|=$ 1. Par exemple, le premier mode correspond à l'époque (*epoch*), le second à la fréquence et le troisième à l'espace (c'est-à-dire aux électrodes).

A l'itération $f$ de la décomposition PARAFAC, on recherche les vecteurs de projection $\mathbf{w}_f^1, \mathbf{w}_f^2, \mathbf{w}_f^3$ qui vérifient :

$$\min_{\underline{\widehat{\mathbf{XY}}}_u} \left\|\underline{\mathbf{XY}}_u - \underline{\widehat{\mathbf{XY}}}_u\right\|^2 \quad \text{avec} \quad \underline{\widehat{\mathbf{XY}}}_u = \theta_f \mathbf{w}_f^1 \circ \mathbf{w}_f^2 \circ \mathbf{w}_f^3 \quad \text{et} \quad \left\|\mathbf{w}_f^1\right\|=\left\|\mathbf{w}_f^2\right\|=\left\|\mathbf{w}_f^3\right\|=1$$

$$(11)$$

Ce problème de minimisation peut être résolu par une méthode des moindres carrés alternés ou ALS (*Alternative*

*Least* Squares) dans laquelle on effectue séquentiellement une minimisation pour chacun des vecteurs de la décomposition, soit :

$$\min_{\mathbf{w}_f^1} \left\| \underline{\mathbf{XY}}_{u(1)} - \mathbf{w}_f^1 \left( \mathbf{w}_f^3 \otimes \mathbf{w}_f^2 \right)^T \right\|^2 \qquad (12\text{-}1)$$

$$\min_{\mathbf{w}_f^2} \left\| \underline{\mathbf{XY}}_{u(2)} - \mathbf{w}_f^2 \left( \mathbf{w}_f^3 \otimes \mathbf{w}_f^1 \right)^T \right\|^2 \qquad (12\text{-}2)$$

$$\min_{\mathbf{w}_f^3} \left\| \underline{\mathbf{XY}}_{u(3)} - \mathbf{w}_f^3 \left( \mathbf{w}_f^2 \otimes \mathbf{w}_f^1 \right)^T \right\|^2 \qquad (12\text{-}3)$$

où $\otimes$ est le produit de Kronecker et $\underline{\mathbf{Z}}_{(n)}$ est une matrice obtenue par déploiement du tenseur $\underline{\mathbf{Z}}$ en prenant le mode $n$ comme mode de référence (*tensor unfolding or flattening along mode n*). Par exemple, la matrice $\underline{\mathbf{Z}}_{(1)}$ est définie par

$$\left( \mathbf{z}_1^1 \big| \ldots \big| \mathbf{z}_1^{I_1} \right) \in \mathbb{R}^{I_1 \times I_2 I_3}$$ où les $\mathbf{z}_1^j$ sont les colonnes de la matrice $\underline{\mathbf{Z}}_{(1)}$.

Les opérations de minimisation (12-1), (12-2) et (12-3) sont répétées jusqu'à la convergence. Les solutions de minimisation au sens des moindres carrés (LS) pour chacune de ces opérations sont données par les projecteurs :

$$\mathbf{w}_f^1 = \frac{\underline{\mathbf{XY}}_{u(1)} \left( \mathbf{w}_f^3 \otimes \mathbf{w}_f^2 \right)}{\left\| \mathbf{w}_f^3 \otimes \mathbf{w}_f^2 \right\|^2} \qquad (13\text{-}1)$$

$$\mathbf{w}_f^2 = \frac{\underline{\mathbf{XY}}_{u(2)} \left( \mathbf{w}_f^3 \otimes \mathbf{w}_f^1 \right)}{\left\| \mathbf{w}_f^3 \otimes \mathbf{w}_f^1 \right\|^2} \qquad (13\text{-}2)$$

$$\mathbf{w}_f^3 = \frac{\underline{\mathbf{XY}}_{u(3)} \left( \mathbf{w}_f^2 \otimes \mathbf{w}_f^1 \right)}{\left\| \mathbf{w}_f^2 \otimes \mathbf{w}_f^1 \right\|^2} \qquad (13\text{-}3)$$

Chaque itération $f$ de la décomposition PARAFAC fournit une pluralité $M$ de projecteurs $\mathbf{w}_f^1, \ldots, \mathbf{w}_f^M$ et un tenseur de coefficients de prédiction, ou modèle prédictif de l'interface neuronale directe, défini par un tenseur de coefficients de prédiction $\underline{\mathbf{B}}_u^f \in \mathbb{R}^{(I_1 \times \ldots \times I_N) \times (J_1 \times \ldots \times J_M)}$ et un tenseur de biais $\underline{\boldsymbol{\beta}}_u^f \in \mathbb{R}^{(J_1 \times \ldots \times J_M)}$, permettant prédire le tenseur de commande à partir du tenseur d'observation. On obtient ainsi une pluralité de modèles, $\underline{\mathbf{B}}_u^f$, $f = 1, \ldots, F$ correspondant à différentes dimensions successives de l'espace des variables latentes.

On détermine ensuite, à l'étape de calibration suivante, u+1, parmi les modèles prédictifs $\underline{\mathbf{B}}_u^f$, $f = 1, \ldots, F,$ le modèle prédictif $\underline{\mathbf{B}}_u^{f^*}$ correspondant à l'erreur de prédiction la plus faible où l'on a noté $f^*$ l'indice $f$ correspondant à cette plus faible erreur de prédiction. Cette détermination du modèle prédictif à l'étape de calibration suivante est désignée dans la littérature relative sous le terme de validation récursive.

En pratique, la méthode REW-NPLS n'est pas appliquée telle quelle sur les données d'observation obtenues pendant l'étape d'apprentissage. En effet, il est préférable d'utiliser un terme de pénalisation dans l'équation de régression de manière à améliorer la robustesse de la prédiction et à accroître la rareté des coefficients non nuls (*sparsity*) dans les vecteurs de pondération $\mathbf{w}_i^f$. En particulier, lorsque le mode est le mode spatial (électrodes), la rareté des coefficients dans ce mode permet de sélectionner seulement les électrodes pertinentes pour la prédiction de la trajectoire.

Il a été ainsi proposé une méthode REW-NPLS pénalisée ou PREW-NPLS ( *Penalized REW-NPLS*) introduisant une pénalisation par tranche à chaque itération de l'algorithme PARAFAC. Une pénalisation par tranche se traduit par une élimination des éléments relatifs à une valeur d'indice dans le tenseur du modèle de prédiction. Les éléments ne faisant pas l'objet d'une pénalisation lors d'une itération $f$ ne sont plus pénalisables pour les itérations suivantes, $f+1$, $f+2$,...,$F$. En revanche les éléments pénalisés lors d'une itération sont pris en compte lors de la décomposition PARAFAC de l'itération suivante. On promeut ainsi une rareté par blocs (*blockwise sparsity*) des éléments non nuls dans le tenseur du modèle de prédiction, ce qui simplifie considérablement le calcul de la commande.

Plus précisément, lors d'une itération $f$ de la décomposition PARAFAC, la minimisation des écarts quadratiques selon (12-1), (12-2) et (12-3) dans la méthode des moindres carrés alternés est remplacée par une minimisation prenant en compte un terme de pénalisation favorisant cette rareté, à savoir :

$$\min_{\tilde{\mathbf{w}}_f^1}\left(\left\|\underline{\mathbf{XY}}_{u(1)}-\tilde{\mathbf{w}}_f^1\left(\mathbf{w}_f^3\otimes\mathbf{w}_f^2\right)^T\right\|^2+\lambda_1\left\|\tilde{\mathbf{w}}_f^1\right\|_{q,\Omega_{1,f}}\right) \qquad (14\text{-}1)$$

$$\min_{\tilde{\mathbf{w}}_f^2}\left(\left\|\underline{\mathbf{XY}}_{u(2)}-\tilde{\mathbf{w}}_f^2\left(\mathbf{w}_f^3\otimes\mathbf{w}_f^1\right)^T\right\|^2+\lambda_2\left\|\tilde{\mathbf{w}}_f^2\right\|_{q,\Omega_{2,f}}\right) \qquad (14\text{-}2)$$

$$\min_{\tilde{\mathbf{w}}_f^3}\left(\left\|\underline{\mathbf{XY}}_{u(3)}-\tilde{\mathbf{w}}_f^3\left(\mathbf{w}_f^2\otimes\mathbf{w}_f^1\right)^T\right\|^2+\lambda_3\left\|\tilde{\mathbf{w}}_f^3\right\|_{q,\Omega_{3,f}}\right) \qquad (14\text{-}3)$$

où $\tilde{\mathbf{w}}_f^1$, $\tilde{\mathbf{w}}_f^2$ et $\tilde{\mathbf{w}}_f^3$ sont des projecteurs non normalisés et $\mathbf{w}_f^1=\dfrac{\tilde{\mathbf{w}}_f^1}{\left\|\tilde{\mathbf{w}}_f^1\right\|}$, $\mathbf{w}_f^2=\dfrac{\tilde{\mathbf{w}}_f^2}{\left\|\tilde{\mathbf{w}}_f^2\right\|}$, $\mathbf{w}_f^3=\dfrac{\tilde{\mathbf{w}}_f^3}{\left\|\tilde{\mathbf{w}}_f^3\right\|}$ sont les projecteurs normalisés correspondants et $\lambda_1,\lambda_2,\lambda_3$ sont des coefficients strictement positifs et inférieurs à 1. . Les normes $\left\|\mathbf{w}_f^i\right\|_{q,\Omega_i}$ $q=0,\dfrac{1}{2},1$ sont définies par :

$$\left\|\mathbf{w}_f^i\right\|_{0,\Omega_{i,f},}=\sum_{k\in\Omega_{i,f}}\left(1-\delta_{0,w_{f,k}^i}\right) \qquad (15\text{-}1)$$

$$\left\|\mathbf{w}_f^i\right\|_{1/2,\Omega_{i,f}}=\sum_{k\in\Omega_{i,f}}\sqrt{\left|w_{f,k}^i\right|} \qquad (15\text{-}2)$$

$$\left\|\mathbf{w}_f^i\right\|_{1,\Omega_{i,f}}=\sum_{k\in\Omega_{i,f}}\left|w_{f,k}^i\right| \qquad (15\text{-}3)$$

où $w_{f,k}^i$ est le $k^{ème}$ élément du vecteur de projection $\mathbf{w}_f^i$, $\delta$ est le symbole de Kronecker, et $\Omega_{i,f}$ est l'ensemble des indices correspondant aux éléments pénalisables, étant compris que $\Omega_{i,f+1} \subset \Omega_{i,f}$ puisque les éléments non-pénalisés d'un projecteur à une itération $f$ ne sont pas pénalisables à l'itération $f+1$.

Afin de simplifier le calcul de minimisation dans les expressions (14-1), (14-2), (14-3), on opère élément par élément, à savoir :

$$\min_{w_{f,k}^1} \left( \left\| \mathbf{z}_1^k - w_{f,k}^1 \left( \mathbf{w}_f^3 \otimes \mathbf{w}_f^2 \right)^T \right\|^2 + \lambda_1 g_q \left( w_{f,k}^1 \right) \right), \ k = 1, ..., I_1 \qquad (16\text{-}1)$$

$$\min_{w_{f,k}^2} \left( \left\| \mathbf{z}_2^k - w_{f,k}^2 \left( \mathbf{w}_f^3 \otimes \mathbf{w}_f^1 \right)^T \right\|^2 + \lambda_2 g_q \left( w_{f,k}^2 \right) \right), \quad k = 1, ..., I_2 \qquad (16\text{-}2)$$

$$\min_{w_{f,k}^3} \left( \left\| \mathbf{z}_3^k - w_{f,k}^3 \left( \mathbf{w}_f^2 \otimes \mathbf{w}_f^1 \right)^T \right\|^2 + \lambda_3 g_q \left( w_{f,k}^3 \right) \right), \ k = 1, ..., I_3 \qquad (16\text{-}3)$$

où les vecteurs $\mathbf{z}_1^k, \mathbf{z}_2^k, \mathbf{z}_3^k$ sont les $k^{èmes}$ vecteurs colonnes des matrices $\underline{\mathbf{XY}}_{u(1)}, \underline{\mathbf{XY}}_{u(2)}, \underline{\mathbf{XY}}_{u(3)}$ obtenues par déploiement du tenseur de covariance croisée $\underline{\mathbf{XY}}_u$ selon les modes 1, 2 et 3, respectivement.

Les fonctions $g_q$, $q = 0, \dfrac{1}{2}, 1$ s'appliquent à des valeurs scalaires et sont définies par :

$$g_0(w_{f,k}^i) = 1 - \delta_{0, w_{f,k}^i} \quad \text{si} \ k \in \Omega_{i,f} \ \text{et} \ g_0(w_{f,k}^i) = 0 \ \text{sinon} \qquad (17\text{-}1)$$

$$g_{1/2}(w_{f,k}^i) = \sqrt{\left| w_{f,k}^i \right|} \quad \text{si} \ k \in \Omega_{i,f} \ \text{et} \ g_{1/2}(w_{f,k}^i) = 0 \ \text{sinon} \qquad (17\text{-}2)$$

$$g_1(w_{f,k}^i) = \left| w_{f,k}^i \right| \quad \text{si} \ k \in \Omega_{i,f} \ \text{et} \ g_1(w_{f,k}^i) = 0 \ \text{sinon} \qquad (17\text{-}3)$$

On peut notamment montrer que le résultat de l'opération de minimisation pénalisée selon pour la norme $L_0$ (c'est-à-dire $q = 0$) donne :

$$\left( w_{f,k}^i \right)_{L0} = 0 \quad \text{si} \ k \in \Omega_{i,f} \ \text{et} \ \left| \left( w_{f,k}^i \right)_{LS} \right| \leq Th_{L0}^i \qquad (18\text{-}1)$$

$$\left( w_{f,k}^i \right)_{L0} = \left( w_{f,k}^i \right)_{LS} \quad \text{sinon} \qquad (18\text{-}2)$$

où $\left( w_{f,k}^i \right)_{LS}$ représente la solution de minimisation au sens des moindres carrés (12-1), (12-2), (12-3), c'est-à-dire :

$$\left(w^1_{f,k}\right)_{LS} = \frac{\mathbf{z}^k_1 \left(\mathbf{w}^3_f \otimes \mathbf{w}^2_f\right)}{\left\|\mathbf{w}^3_f \otimes \mathbf{w}^2_f\right\|^2} \tag{19-1}$$

$$\left(w^2_{f,k}\right)_{LS} = \frac{\mathbf{z}^k_2 \left(\mathbf{w}^3_f \otimes \mathbf{w}^1_f\right)}{\left\|\mathbf{w}^3_f \otimes \mathbf{w}^1_f\right\|^2} \tag{19-2}$$

$$\left(w^3_{f,k}\right)_{LS} = \frac{\mathbf{z}^k_3 \left(\mathbf{w}^2_f \otimes \mathbf{w}^1_f\right)}{\left\|\mathbf{w}^2_f \otimes \mathbf{w}^1_f\right\|^2} \tag{19-3}$$

et les seuils $Th^i_{L0}$, $i = 1,2,3$ sont donnés par :

$$Th^1_{L0} = \frac{\sqrt{\lambda_1}}{\left\|\mathbf{w}^3_f \otimes \mathbf{w}^2_f\right\|} \tag{20-1}$$

$$Th^2_{L0} = \frac{\sqrt{\lambda_2}}{\left\|\mathbf{w}^3_f \otimes \mathbf{w}^1_f\right\|} \tag{20-2}$$

$$Th^3_{L0} = \frac{\sqrt{\lambda_3}}{\left\|\mathbf{w}^2_f \otimes \mathbf{w}^1_f\right\|} \tag{20-3}$$

De manière similaire, d'autres valeurs de seuil peuvent être calculées pour les normes $L_{1/2}$ et $L_1$.

On comprend que la minimisation pénalisée revient par conséquent à effectuer un seuillage sur les éléments des projecteurs obtenus à chaque étape de la méthode ALS.

On pourra trouver une description détaillée de la méthode PREW-NPLS dans l'article de A. Moly et al. intitulé « On-line adaptive group-wise sparse NPLS for ECoG neural signal decoding », publié le 14.10.2020 sur arXiv.org ainsi que dans la demande française FR2010497 déposée le 14.10.2020 au nom de la présente demanderesse.

Les seuils précités, $Th^i_{Lq}$, dépendent des valeurs des hyperparamètres $\lambda_i$ (coefficients de pénalisation). Le choix de ces hyperparamètres est critique pour déterminer le degré de rareté par blocs des éléments non nuls du modèle prédictif. Tout comme la dimension de l'espace des variables latentes, $F$, les valeurs des hyperparamètres $\lambda_i$ sont généralement optimisées au moyen d'une phase de validation croisée entre un jeu de données d'entrainement et un jeu de données de test. Cette optimisation nécessite toutefois de prendre en compte à chaque fois l'ensemble complet des données disponibles et requiert par conséquent des calculs importants qui ne sont pas compatibles avec un apprentissage en ligne.

L'objet de la présente invention est par conséquent de proposer une interface neuronale directe dont le modèle prédictif fait l'objet d'une calibration en ligne multivoie pénalisée, et plus particulièrement d'une calibration PREW-NPLS, dans laquelle l'adaptation des coefficients de pénalisation est réalisée de manière adaptative au cours de l'apprentissage sans nécessiter de calculs importants.

La publication d'Alexandre Moly et al., "Online adaptive group-wise sparse NPLS for ECoG neural signal decoding", ARXIV.ORG, CORNELL UNIVERSITY LIBRARY, 201 OLIN LIBRARY CORNELL UNIVERSITY ITHACA, NY 14853, 14 octobre 2020 (2020-10-14), XP081791002, fournit une présentation de l'arrière-plan technologique de l'invention.

**EXPOSÉ DE L'INVENTION**

La présente invention est définie par une méthode de calibration en ligne d'une interface neuronale directe destinée à recevoir une pluralité de signaux électrophysiologiques pendant une pluralité de fenêtres d'observation associées à des instants d'observation, pour former un tenseur d'entrée et en déduire, au moyen d'un modèle prédictif représenté par un tenseur de prédiction, un tenseur de sortie fournissant des signaux de commande destinés à commander un ou plusieurs effecteurs, ladite calibration étant effectuée lors d'une pluralité de phases de calibration, chaque phase de calibration *u* étant réalisée à partir de données de calibration d'entrée, représentées par un tenseur de calibration

d'entrée, $\underline{\mathbf{X}}_u \in \mathbb{R}^{(I_1 \times ... \times I_N)}$, et de données de calibration de sortie représentées par un tenseur de calibration de sortie

$\underline{\mathbf{Y}}_u \in \mathbb{R}^{(J_1 \times ... \times J_M)}$, ladite phase de calibration mettant en oeuvre une régression REW-NPLS pénalisée, la pénalisation portant sur au moins une modalité du tenseur de prédiction et faisant intervenir au moins un hyperparamètre, ladite méthode étant originale en ce que, lors d'une phase de calibration :

- on extrait un sous-ensemble, $\Lambda_u^{test}$, de valeurs de l'hyperparamètre à tester d'un ensemble, $\Lambda$, de valeurs de l'hyperparamètre ;

- on calcule un indicateur de performance de prédiction ( $e_u^{\lambda,f}$ ) pour chaque modèle prédictif d'une pluralité de modèles prédictifs pénalisés, chaque modèle prédictif pénalisé ( $\boldsymbol{\Theta}_{u-1}^{\lambda,f}$ ) étant associé à une dimension (*f*) de l'espace des variables latentes et ayant été obtenu, lors de la phase de calibration précédente, *u* - 1, par régression REW-NPLS pénalisée au moyen d'une valeur d'hyperparamètre ( $\lambda$ ) dudit sous-ensemble, l'indicateur de prédiction du modèle prédictif pénalisé représentant la qualité de la prédiction du tenseur de calibration de sortie par ce modèle à partir du tenseur de calibration d'entrée

- on calcule pour chaque valeur d'hyperparamètre ( $\lambda$ ) dudit sous-ensemble, une récompense ( $R_u^{\lambda}$ ) associée au choix de cette valeur , à partir des indicateurs de performance de prédiction ( $e_u^{\lambda,f}$ ) des modèles prédictifs pénalisés ( $\boldsymbol{\Theta}_{u-1}^{\lambda,f}$ ) ;

- pour chaque valeur d'hyperparamètre ( $\lambda$ ) dudit sous-ensemble, on met à jour (140) une valeur d'action ( $Q_u^{\lambda}$ ) correspondant au choix de cette valeur, à partir de la récompense obtenue ( $R_u^{\lambda}$ ) pour le choix de cette valeur à l'étape précédente ;
- on sélectionne selon une stratégie de sélection d'action une valeur d'hyperparamètre ( $\lambda^*$ ) parmi l'ensemble $\Lambda$ des valeurs d'hyperparamètre à partir des valeurs d'action ainsi mises à jour ( $Q_u^{\lambda}$ );
- on met à jour chacun des modèles prédictifs pénalisés de ladite pluralité, au moyen d'une régression REW-NPLS pénalisée au moyen d'une valeur d'hyperparamètre ( $\lambda$ ) de l'ensemble $\Lambda$ ;
- on utilise le modèle prédictif pénalisé au moyen de ladite valeur d'hyperparamètre sélectionnée et mis à jour à l'étape précédente, pour prédire un tenseur de commande fournissant lesdits signaux de commande à partir dudit tenseur d'entrée

Avantageusement, l'indicateur de performance de prédiction, $e_u^{\lambda,f}$, d'un modèle prédictif pénalisé associé à une dimension *f* de l'espace des variables latentes et à une valeur d'hyperparamètre $\lambda$ du sous-ensemble est obtenu

$$e_u^{\lambda,f} = \mu . e_{u-1}^{\lambda,f} + \delta \left( \underline{\mathbf{Y}}_u , \widehat{\underline{\mathbf{Y}}}_u^{\lambda,f} \right)$$

de manière récursive par où $\widehat{\underline{\mathbf{Y}}}_u^{\lambda,f}$ est la prédiction de tenseur de sortie obtenue à partir du modèle prédictif pénalisé obtenu lors la phase de calibration précédente, $\boldsymbol{\Theta}_{u-1}^{\lambda,f}$, $\delta\left(\underline{\mathbf{Y}}_u, \widehat{\underline{\mathbf{Y}}}_u^{\lambda,f}\right)$ est une fonction décroissante de l'écart entre les tenseurs $\underline{\mathbf{Y}}_u$ et $\widehat{\underline{\mathbf{Y}}}_u^{\lambda,f}$, et $0 < \mu < 1$ est un coefficient d'oubli.

La récompense $R_u^{\lambda}$ associée au choix de la valeur d'hyperparamètre $\lambda$, peut alors être obtenue à partir du meilleur indicateur de performance de prédiction, $e_u^{\lambda^*,f}$, obtenue par les modèles prédictifs pénalisés $\boldsymbol{\Theta}_{u-1}^{\lambda,f}$, sur la pluralité des dimensions possibles, $f = 1,...,F$ de l'espace des variables latentes.

La récompense $R_u^{\lambda}$ peut alors être donnée par $$R_u^{\lambda} = \max_{\lambda \in \Lambda}\left(rank\left(e_u^{\lambda}\right)\right) - rank\left(e_u^{\lambda}\right)$$ où $rank(.)$ est une fonction de tri par valeurs décroissantes.

Selon une variante, la récompense ( $R_u^{\lambda}$ ) associée au choix d'une valeur d'hyperparamètre ( $\lambda$ ) est obtenue en outre à partir d'indicateurs de parcimonie des modèles prédictifs pénalisés ( $\boldsymbol{\Theta}_{u-1}^{\lambda,f}$ ), un indicateur de parcimonie d'un modèle prédictif pénalisé étant définie comme une fonction croissante du nombre d'éléments nuls du tenseur de prédiction ( $\mathbf{B}_{u-1}^{\lambda,f}$ ) qui le représente.

Selon un premier exemple de réalisation, la valeur d'action, $Q_u^{\lambda}$, associée au choix d'une valeur d'hyperpa-ramètre, $\lambda$, à la phase de calibration $u$ est obtenue par $$Q_u^{\lambda} = Q_{u-1}^{\lambda} + \frac{1}{u}\left(R_u^{\lambda} - Q_{u-1}^{\lambda}\right)$$, où $Q_{u-1}^{\lambda}$ est la valeur d'action associée à ce choix, lors de la phase de calibration précédente, $R_u^{\lambda}$ est la récompense associée à ce même choix et $u$ est le nombre de phases de calibration effectuées.

Selon un second exemple de réalisation, la valeur d'action, $Q_u^{\lambda}$, associée au choix d'une valeur d'hyperpa-ramètre, $\lambda$, à la phase de calibration $u$ est obtenue par $$Q_u^{\lambda} = Q_{u-1}^{\lambda} + \eta\left(R_u^{\lambda} - Q_{u-1}^{\lambda}\right)$$, où $Q_{u-1}^{\lambda}$ est la valeur d'action associée à ce choix, lors de la phase de calibration précédente, $R_u^{\lambda}$ est la récompense associée à ce même choix et $\eta$ est un facteur de mise à jour avec $0 < \eta < 1$.

Selon un premier mode de réalisation, la valeur d'hyperparamètre ($\lambda^*$) sélectionnée est obtenue au moyen d'une stratégie gloutonne comme celle correspondant à la valeur d'action la plus élevée sur l'ensemble des valeurs d'hyperparamètre ($\Lambda$).

Selon un second mode de réalisation, la valeur d'hyperparamètre sélectionnée ($\lambda^*$) est obtenue au moyen d'une stratégie $\varepsilon$ - gloutonne, cette valeur étant prise avec une probabilité $\varepsilon$ comme celle correspondant à la valeur d'action la plus élevée sur l'ensemble des valeurs d'hyperparamètre ($\Lambda$) et avec une probabilité ($1$-$\varepsilon$) parmi les autres valeurs, dites sous-optimales.

Avantageusement, avant l'étape de sélection de la valeur d'hyperparamètre lors de la phase de calibration $u$, chaque valeur d'action, $Q_u^{\lambda}$, associée au choix d'une valeur d'hyperparamètre sous-optimale, $\lambda$, est majorée par $$Q'_u^{\lambda} = Q_{u-1}^{\lambda} + \theta.\frac{u - u(\lambda)}{u}$$ où $u(\lambda)$ est l'indice de la dernière phase de calibration dans laquelle la valeur $\lambda$ a été sélectionnée et $\theta$ est un paramètre positif non nul.

## BRÈVE DESCRIPTION DES DESSINS

D'autres caractéristiques et avantages de l'invention apparaîtront à la lecture d'un mode de réalisation préférentiel de l'invention, en faisant référence aux figures jointes parmi lesquelles :

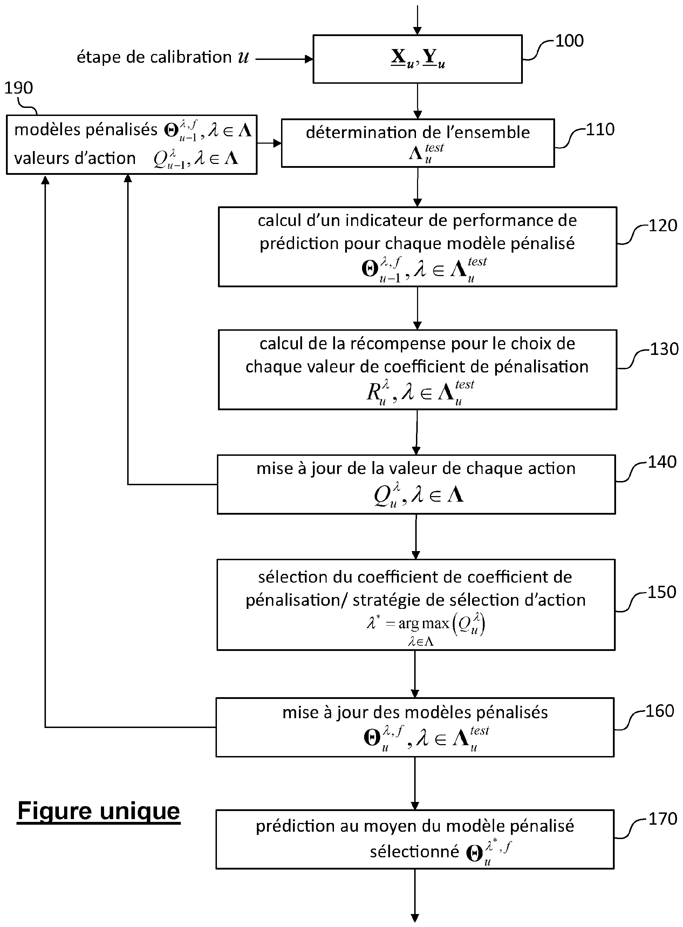

La Figure unique représente schématiquement une méthode de calibration en ligne d'une interface neuronale directe selon un mode de réalisation de la présente invention.

## EXPOSÉ DÉTAILLÉ DE MODES DE RÉALISATION PARTICULIERS

Nous nous placerons dans la suite dans le cas d'une interface neuronale directe (BCI), et considérerons plus particulièrement la phase de calibration (ou de manière équivalente d'apprentissage) d'une telle interface. Le système d'acquisition des signaux électrophysiologiques peut être un système ECoG ou un système ECG.

Les phases de calibration interviennent en ligne, c'est-à-dire alors que l'interface BCI est opérationnelle.

Lors d'une phase de calibration le sujet imagine ou réalise un mouvement selon une trajectoire prédéterminée ou enregistrée pendant un intervalle donné. Les signaux électrophysiologiques acquis par l'interface pendant la phase de calibration font l'objet d'un prétraitement avant d'être utilisés pour l'estimation des paramètres du modèle prédictif. De manière connue en soi, le prétraitement peut comprendre par exemple un centrage et une normalisation des signaux, une analyse temps-fréquence, un calcul de valeur absolue ou de valeur quadratique des coefficients résultant de cette analyse, optionnellement un lissage fréquentiel ou une interpolation (calcul d'enveloppe) des valeurs ainsi obtenues.

A chaque phase de calibration u, on dispose ainsi d'un couple de tenseurs $\underline{\mathbf{X}}_u, \underline{\mathbf{Y}}_u$ correspondant respectivement aux observations des signaux électrophysiologiques (variables d'entrée) et des signaux de commande (variables de sortie), tels que ceux d'un robot multi-axe par exemple. La phase de calibration vise à obtenir les paramètres d'un modèle prédictif linéaire permettant de prédire le tenseur de variables de sortie à partie du tenseur de variables d'entrée.

L'idée à la base de l'invention est d'utiliser une méthode de calibration REW-NPLS pénalisée (PREW-NPLS) dans laquelle les hyperparamètres intervenant la pénalisation, notamment les coefficients de pénalisation sont obtenus au moyen d'un apprentissage par renforcement. Plus précisément, la sélection des valeurs des hyperparamètres est réalisée au moyen d'un algorithme de bandit-manchot multi-bras (*multi-armed bandit*). La sélection des valeurs des hyperparamètres intervenant dans la pénalisation est réalisée lors de l'étape de validation récursive de la méthode REW-NPLS. Autrement dit, cette étape de validation récursive sert désormais à la sélection de l'ensemble des hyperparamètres, que ce soit la dimension de l'espace des variables latentes ou les coefficients de pénalisation.

La Figure unique représente de manière schématique l'ordinogramme d'une méthode de calibration en ligne d'une interface neuronale directe selon un mode de réalisation de la présente invention.

Cet ordinogramme décrit le déroulement d'une telle phase de calibration, cette phase débutant, à l'étape 100, par la prise en compte de nouvelles données de calibration d'entrée et de sortie, respectivement sous la forme d'un tenseur d'entrée $\underline{\mathbf{X}}_u$ et d'un tenseur de sortie $\underline{\mathbf{Y}}_u$.

A ce stade, on dispose également en mémoire des paramètres des modèles prédictifs pénalisés tels qu'estimés lors de la phase de calibration précédente, $u$-1.

On notera dans la suite $\Lambda$ l'ensemble des coefficients de pénalisation intervenant dans l'estimation du modèle prédictif pénalisé. De manière générale, cet ensemble peut être constitué de L-uplets de valeurs discrètes et ou d'intervalles de variation de ces coefficients. Sans perte de généralité, nous nous placerons dans la première hypothèse dans un but d'illustration. Ainsi, dans l'exemple de modèle prédictif cité dans la partie introductive, l'ensemble $\Lambda$ pourra être constitué valeurs discrètes ( $\lambda_1^\ell, \lambda_2^\ell, \lambda_3^\ell$ ). Un élément de $\Lambda$ sera noté de manière générique $\lambda$. Dans la suite, on pourra supposer que $L = 1$ par souci de simplification. Ce sera notamment le cas si la pénalisation ne porte que sur le mode spatial (électrodes).

Un modèle prédictif pénalisé par le coefficient de pénalisation $\lambda$, obtenu à l'issue d'une phase de calibration $u$ et pour une dimension $f$ de l'espace des variables latentes sera noté dans la suite $\Theta_u^{\lambda,f} = \left( \underline{\mathbf{B}}_u^{\lambda,f}, \underline{\boldsymbol{\beta}}_u^{\lambda,f} \right)$. L'ensemble des modèles prédictifs pénalisés associés aux différentes dimensions possibles de l'espace des variables latentes, $f = 1,...,F$ sera noté $\Theta_u^{\lambda}$. Autrement dit, $\Theta_u^{\lambda} = \left\{ \Theta_u^{\lambda,f}; f = 1,...,F \right\}$.

Ainsi, lors de la phase de calibration u, on dispose en mémoire, 190, de l'ensemble des modèles pénalisés $\Theta_{u-1}^{\lambda}$, $\lambda \in \Lambda$. En outre, pour chaque valeur de $\lambda \in \Lambda$, la mémoire contient une valeur d'action (*action value*), $Q_{u-1}^{\lambda}$, représentant, au sens de l'apprentissage par renforcement, l'estimation de récompense attendue (*expected reward*) en réponse à la sélection de l'action (ici la sélection de la valeur $\lambda$), compte tenu des récompenses passées $R_1^{\lambda},...,R_{u-1}^{\lambda}$

lors des phases de calibration précédentes. La récompense $R_u^{\lambda}$ associée à la sélection de la valeur de l'hyperparamètre $\lambda$ lors de la phase de calibration $u$ sera précisée plus loin.

A l'étape 110, on construit un sous-ensemble $\mathbf{\Lambda}_u^{test} \subset \mathbf{\Lambda}$ de valeurs de coefficients de pénalisation (hyperparamètres à tester lors de la phase de calibration $u$), dit ensemble de test, à partir de l'ensemble de test, $\mathbf{\Lambda}_{u-1}^{test}$ utilisé lors de la phase de calibration précédente et d'autres valeurs de coefficients de pénalisation appartenant à $\mathbf{\Lambda} - \mathbf{\Lambda}_{u-1}^{test}$ choisies selon un critère prédéterminé.

Selon une première variante de réalisation, les autres valeurs de coefficients de pénalisation sont obtenues par tirage aléatoire dans l'ensemble $\mathbf{\Lambda} - \mathbf{\Lambda}_{u-1}^{test}$. Selon une seconde variante, les autres valeurs de coefficients de pénalisation sont obtenues en sélectionnant dans l'ensemble $\mathbf{\Lambda} - \mathbf{\Lambda}_{u-1}^{test}$ celles qui correspondent aux valeurs d'action $Q_{u-1}^{\lambda}$ les plus élevées.

A l'étape 120, on calcule un indicateur de performance de prédiction pour chaque modèle prédictif pénalisé, $\mathbf{\Theta}_{u-1}^{\lambda,f}$, ce pour chaque coefficient de pénalisation de l'ensemble de test $\lambda \in \mathbf{\Lambda}_u^{test}$, et pour chaque dimension $f \in \{1,...,F\}$ de l'espace des variables latentes.

En d'autres termes, on détermine dans quelle mesure les modèles prédictifs obtenus lors de la phase de calibration précédente peuvent permettre de prédire le tenseur de sortie $\underline{\mathbf{Y}}_u$ à partir du tenseur d'entrée $\underline{\mathbf{X}}_u$.

L'indicateur de performance de prédiction, $e_u^{\lambda,f}$, du modèle pénalisé peut être déterminé de manière itérative :

$$e_u^{\lambda,f} = \mu.e_{u-1}^{\lambda,f} + \delta\left(\underline{\mathbf{Y}}_u, \widehat{\underline{\mathbf{Y}}}_u^{\lambda,f}\right), \quad \lambda \in \mathbf{\Lambda}_u^{test}, \quad f = 1,...,F \tag{21}$$

où $\widehat{\underline{\mathbf{Y}}}_u^{\lambda,f} = \underline{\mathbf{X}}_t \underline{\mathbf{B}}_{u-1}^{\lambda,f} + \underline{\mathbf{\beta}}_{u-1}^{\lambda,f}$ est la prédiction de tenseur de sortie obtenue à partir du modèle prédictif pénalisé obtenu lors la phase de calibration précédente, $\mathbf{\Theta}_{u-1}^{\lambda,f}$, et $\delta\left(\underline{\mathbf{Y}}_u, \widehat{\underline{\mathbf{Y}}}_u^{\lambda,f}\right)$ est une fonction décroissante de l'écart entre les tenseurs $\underline{\mathbf{Y}}_u$ et $\widehat{\underline{\mathbf{Y}}}_u^{\lambda,f}$, et $0 < \mu < 1$ est un coefficient d'oubli.

En revanche, l'indicateur de performance d'un modèle de prédiction non testé est laissé inchangé, c'est-à-dire :

$$e_u^{\lambda,f} = e_{u-1}^{\lambda,f}, \quad \lambda \in \mathbf{\Lambda} - \mathbf{\Lambda}_u^{test}, \quad f = 1,...,F \tag{22}$$

On estime ensuite à l'étape 130, la récompense associée à la sélection de chaque valeur $\lambda \in \mathbf{\Lambda}_u^{test}$ du coefficient de pénalisation. Plus précisément, cette récompense est obtenue à partir des indicateurs de performance $e_u^{\lambda,f}$ des modèles de prédiction $\mathbf{\Theta}_{u-1}^{\lambda,f}$.

Pour ce faire, on détermine d'abord la dimension de l'espace des variables latentes permettant d'obtenir la meilleure prédiction, soit :

$$f^* = \arg\max_{f \in \{1,...,F\}} \left( e_u^{\lambda,f} \right) \tag{23}$$

et l'on en déduit l'indicateur de performance maximale sur les valeurs de *f*, noté $e_u^{\lambda} = e_u^{\lambda,f^*}$ .

La récompense, $R_u^{\lambda}$ , pour la sélection de la valeur $\lambda \in \Lambda$ lors de la phase de calibration *u* est déterminée à partir de l'indicateur de performance maximale relatif à cette valeur, par exemple :

$$R_u^{\lambda} = \max_{\lambda \in \Lambda} \left( rank \left( e_u^{\lambda} \right) \right) - rank \left( e_u^{\lambda} \right) \tag{24}$$

où *rank*(.) est une fonction de tri par valeurs décroissantes. Autrement dit, la valeur de l'hyperparamètre $\lambda$ associée à l'indicateur de performance maximale, $e_u^{\lambda}$ , le plus élevé se voit attribuer le premier rang (égal à 1) et celle associée à l'indicateur de performance maximale le moins élevé se voir attribuer le dernier rang (égal à $\max_{\lambda \in \Lambda} \left( rank \left( e_u^{\lambda} \right) \right)$ ).

L'homme du métier comprendra que d'autres expressions de la fonction de récompense $R_u^{\lambda}$ pourront être envisagées sans sortir pour autant du cadre de la présente invention. En particulier, la récompense obtenue pourra non seulement dépendre d'un indicateur de performance de prédiction (plus précisément de sa valeur maximale prise sur l'ensemble *f* = 1,...,*F*) mais également d'un indicateur de parcimonie du tenseur $\underline{\mathbf{B}}_u^{\lambda,f}$ , $s_u^{\lambda,f}$ , fonction croissante du nombre d'éléments nuls de ce tenseur. Comme précédemment l'indicateur de parcimonie pourra être obtenu de manière récursive sur l'indice de la phase de calibration, par analogie avec l'expression (21).

A l'étape 140, on met à jour la valeur de chaque action stockée en mémoire à partir de la récompense obtenue pour cette action à l'étape précédente :

$$Q_u^{\lambda} = Q_{u-1}^{\lambda} + \frac{1}{u} \left( R_u^{\lambda} - Q_{u-1}^{\lambda} \right) \tag{25}$$

Cette mise à jour suppose que la distribution de probabilité de récompense pour chaque action est stationnaire. En revanche, si cette distribution de probabilité n'est pas stationnaire, on pourra utiliser une mise à jour donnant plus d'importance aux récompenses récentes, par exemple :

$$Q_u^{\lambda} = Q_{u-1}^{\lambda} + \eta \left( R_u^{\lambda} - Q_{u-1}^{\lambda} \right) \tag{26}$$

où $0 < \eta < 1$ est un facteur de mise à jour (*step size parameter*) constant. La valeur de l'action n'est autre qu'une moyenne pondérée des récompenses passées :

$$Q_u^{\lambda} = \left( 1 - \eta \right)^u Q_1^{\lambda} + \sum_{i=1}^{u} \eta \left( 1 - \eta \right)^{u-i} R_u^{\lambda} \tag{27}$$

Il convient de noter que cette étape de mise à jour concerne aussi bien les actions associées aux valeurs testées ( $\lambda \in \Lambda_u^{test}$ ) lors de la phase de calibration en cours que celles qui sont associées à des valeurs non testées.

On obtient ainsi pour chaque action, ou de manière équivalente pour chaque valeur d'hyperparamètre, une valeur d'action (*action value*), c'est-à-dire une récompense attendue (*expected reward*), $Q_u^\lambda$ .

On sélectionne ensuite à l'étape 150, la valeur de l'hyperparamètre pour la nouvelle action. Autrement dit, on choisit le coefficient de pénalisation (et de manière plus générale le L-uplet de coefficients de pénalisation) permettant d'espérer obtenir la récompense la plus élevée compte tenu des actions effectuées précédemment, soit :

$$\lambda^* = \arg\max\left(Q_u^\lambda\right) \qquad (28)$$

Cette stratégie, basée exclusivement sur l'exploitation de l'action dont la récompense attendue est la plus élevée, est dite gloutonne (*greedy*). Alternativement on pourra opter pour une stratégie dite $\varepsilon$-gloutonne ($\varepsilon$-*greedy*) résultant d'un compromis entre exploitation de cette action et exploration d'autres actions moins fréquemment prises. En effet, il est possible qu'une action moins explorée ou qui n'a pas été explorée récemment, conduise en définitive à une récompense plus élevée que celle obtenue par une stratégie gloutonne.

Selon une stratégie $\varepsilon$-gloutonne, on choisit l'action optimale, c'est-à-dire correspondant à la valeur (la récompense attendue) la plus élevée avec une probabilité $\varepsilon$ et une action sous-optimale par tirage au sort de manière équiprobable parmi les actions restantes, avec une probabilité globale de 1 - $\varepsilon$. On comprendra ainsi que la valeur $\varepsilon$ permet de régler le compromis entre exploitation et exploration.

Avantageusement, si l'on opte pour une stratégie $\varepsilon$-gloutonne, on pourra sélectionner les actions sous-optimales en privilégiant celles qui n'ont pas été sélectionnées depuis longtemps. Pour ce faire, on peut modifier les valeurs des actions estimées en 140, avant l'étape de sélection 150, comme suit :

$$Q_u^{'\lambda} = Q_{u-1}^\lambda + \theta.\frac{u - u(\lambda)}{u} \qquad (29)$$

où $u(\lambda)$ est l'indice de la dernière phase de calibration dans laquelle la valeur $\lambda$ a été sélectionnée et $\theta$, est un paramètre (réel positif non nul) permettant d'ajuster le compromis entre exploration et exploitation. Autrement dit, lorsque la valeur $\lambda$ a été sélectionnée il y a longtemps, la valeur d'action est majorée. La sélection de la valeur de l'hyperparamètre $\lambda^*$ à l'étape 150 est basée sur les valeurs d'action modifiées, $Q_u^{'\lambda}$ , $\lambda \in \Lambda$.

A l'étape 160, on met à jour chacun des modèles pénalisés $\mathbf{\Theta}_u^{\lambda,f}, \lambda \in \mathbf{\Lambda}_u^{test}$ au moyen de l'algorithme PREW-NPLS, les paramètres des autres modèles pénalisés $\mathbf{\Theta}_u^{\lambda,f}, \lambda \in \mathbf{\Lambda} - \mathbf{\Lambda}_u^{test}$ restant inchangés.

Parmi les modèles pénalisés mis à jour, celui correspondant à la valeur d'hyperparamètre sélectionnée à l'étape 150 est utilisé pour la prédiction du tenseur de sortie dans la phase opérationnelle en 170.

Dans la description précédente, on a supposé que $\Lambda$ était constitué de valeurs discrètes d'un coefficient de pénalisation. De manière plus générale, cet ensemble peut être constitué de *L*-uplets de valeurs discrètes pour *L* coefficients de pénalisation respectivement appliqués à *L* modalités du tenseur de prédiction.

En outre, l'ensemble $\Lambda$ peut être un intervalle de variation [$\lambda_{min}, \lambda_{max}$] d'un coefficient de pénalisation voire un produit cartésien de *L* intervalles dans cas où la pénalisation s'applique à *L* modalités du tenseur de prédiction.

Dans ce cas, on pourra extraire une pluralité de valeurs discrètes dans l'intervalle de variation et mettre à jour chacune d'entre elles au moyen d'un algorithme de plus forte pente (*gradient descent*) pour minimiser l'indicateur de performance de prédiction des modèles pénalisés associés. La mise à jour peut être effectuée à partir des récompenses obtenues par la sélection de ces valeurs discrètes.

Enfin, la méthode d'apprentissage par renforcement des hyperparamètres de pénalisation a été décrite jusqu'ici en relation avec les coefficients de pénalisation s'appliquant sur une ou plusieurs modalités du tenseur de prédiction. Elle peut alternativement ou cumulativement prendre en compte le type de pénalisation, notamment le type de norme $L_q$ ($q$ = 0,1/2,1), la valeur de $q$ étant alors considérée comme un hyperparamètre du modèle.

**Revendications**

1. Méthode de calibration en ligne d'une interface neuronale directe destinée à recevoir une pluralité de signaux électrophysiologiques pendant une pluralité de fenêtres d'observation associées à des instants d'observation, pour former un tenseur d'entrée et en déduire, au moyen d'un modèle prédictif représenté par un tenseur de prédiction, un tenseur de sortie fournissant des signaux de commande destinés à commander un ou plusieurs effecteurs, ladite calibration étant effectuée lors d'une pluralité de phases de calibration, chaque phase de calibration $u$ étant réalisée à partir de données de calibration d'entrée, représentées par un tenseur de calibration d'entrée, $\underline{\mathbf{X}}_u \in \mathbb{R}^{(I_1 \times \ldots \times I_N)}$

, et de données de calibration de sortie représentées par un tenseur de calibration de sortie $\underline{\mathbf{Y}}_u \in \mathbb{R}^{(J_1 \times \ldots \times J_M)}$,
ladite phase de calibration mettant en oeuvre une régression REW-NPLS pénalisée, une régression REW-NPLS, de l'Anglais *'Recursive Exponentially Weigthed N-way Partial Least Squares',* étant une méthode de régression par moindres carrés partiels récursive, multivariée et à pondération exponentielle, la pénalisation portant sur au moins une modalité du tenseur de prédiction et faisant intervenir au moins un hyperparamètre, **caractérisée en ce que**, lors d'une phase de calibration :

- on extrait (110) un sous-ensemble, $\boldsymbol{\Lambda}_u^{test}$, de valeurs de l'hyperparamètre à tester d'un ensemble, $\Lambda$, de valeurs de l'hyperparamètre ;

- on calcule (120) un indicateur de performance de prédiction ( $e_u^{\lambda,f}$ ) pour chaque modèle prédictif d'une pluralité de modèles prédictifs pénalisés, chaque modèle prédictif pénalisé ( $\boldsymbol{\Theta}_{u-1}^{\lambda,f}$ ) étant associé à une dimension ($f$) de l'espace des variables latentes et ayant été obtenu, lors de la phase de calibration précédente, $u$-1, par régression REW-NPLS pénalisée au moyen d'une valeur d'hyperparamètre ($\lambda$) dudit sous-ensemble, l'indicateur de prédiction du modèle prédictif pénalisé représentant la qualité de la prédiction du tenseur de calibration de sortie par ce modèle à partir du tenseur de calibration d'entrée ;

- on calcule (130) pour chaque valeur d'hyperparamètre ($\lambda$) dudit sous-ensemble, une récompense ( $R_u^{\lambda}$ )

associée au choix de cette valeur , à partir des indicateurs de performance de prédiction ( $e_u^{\lambda,f}$ ) des modèles

prédictifs pénalisés ( $\boldsymbol{\Theta}_{u-1}^{\lambda,f}$ ) ;

- pour chaque valeur d'hyperparamètre ($\lambda$) dudit sous-ensemble, on met à jour (140) une valeur d'action ( $Q_u^{\lambda}$

) correspondant au choix de cette valeur, à partir de la récompense obtenue ( $R_u^{\lambda}$ ) pour le choix de cette valeur à l'étape précédente ;
- on sélectionne (150) selon une stratégie de sélection d'action une valeur d'hyperparamètre ($\lambda$*) parmi l'ensemble $\Lambda$ des valeurs d'hyperparamètre à partir des valeurs d'action ainsi mises à jour ( $Q_u^{\lambda}$ );
- on met à jour (160) chacun des modèles prédictifs pénalisés de ladite pluralité, au moyen d'une régression REW-NPLS pénalisée au moyen d'une valeur d'hyperparamètre ($\lambda$) de l'ensemble $\Lambda$ ;
- on utilise (170) le modèle prédictif pénalisé au moyen de ladite valeur d'hyperparamètre sélectionnée et mis à jour à l'étape précédente, pour prédire un tenseur de commande fournissant lesdits signaux de commande à partir dudit tenseur d'entrée.

2. Méthode de calibration en ligne d'une interface neuronale directe selon la revendication 1, **caractérisée en ce que** l'indicateur de performance de prédiction, $e_u^{\lambda,f}$ , d'un modèle prédictif pénalisé associé à une dimension $f$ de l'espace des variables latentes et à une valeur d'hyperparamètre $\lambda$ du sous-ensemble est obtenu de manière

$$e_u^{\lambda,f} = \mu.e_{u-1}^{\lambda,f} + \delta\left(\underline{\mathbf{Y}}_u, \underline{\widehat{\mathbf{Y}}}_u^{\lambda,f}\right)$$

récursive par $\qquad$ où $\underline{\widehat{\mathbf{Y}}}_u^{\lambda,f}$ est la prédiction de tenseur de sortie obtenue à partir du modèle prédictif pénalisé obtenu lors la phase de calibration précédente, $\boldsymbol{\Theta}_{u-1}^{\lambda,f}$, $\delta\left(\underline{\mathbf{Y}}_u, \underline{\widehat{\mathbf{Y}}}_u^{\lambda,f}\right)$ est une

fonction décroissante de l'écart entre les tenseurs $\underline{\mathbf{Y}}_u$ et $\underline{\widehat{\mathbf{Y}}}_u^{\lambda,f}$, et $0 < \mu < 1$ est un coefficient d'oubli.

3. Méthode de calibration en ligne d'une interface neuronale directe selon la revendication 1 ou 2, **caractérisée en ce que** la récompense $R_u^{\lambda}$ associée au choix de la valeur d'hyperparamètre $\lambda$, est obtenue à partir du meilleur indicateur de performance de prédiction, $e_u^{\lambda^*,f}$, obtenue par les modèles prédictifs pénalisés $\boldsymbol{\Theta}_{u-1}^{\lambda,f}$, sur la pluralité des dimensions possibles, $f = 1,...,F$ de l'espace des variables latentes.

4. Méthode de calibration en ligne d'une interface neuronale directe selon la revendication 3, **caractérisée en ce que**

$$R_u^{\lambda} = \max_{\lambda \in \boldsymbol{\Lambda}}\left(rank\left(e_u^{\lambda}\right)\right) - rank\left(e_u^{\lambda}\right)$$

la récompense $R_u^{\lambda}$ est donnée par $\qquad$ où $rank(.)$ est une fonction de tri par valeurs décroissantes.

5. Méthode de calibration en ligne d'une interface neuronale directe selon la revendication 1 ou 2, **caractérisée en ce que** la récompense ($R_u^{\lambda}$) associée au choix d'une valeur d'hyperparamètre ($\lambda$) est obtenue en outre à partir d'indicateurs de parcimonie des modèles prédictifs pénalisés ($\boldsymbol{\Theta}_{u-1}^{\lambda,f}$), un indicateur de parcimonie d'un modèle prédictif pénalisé étant définie comme une fonction croissante du nombre d'éléments nuls du tenseur de prédiction ($\underline{\mathbf{B}}_{u-1}^{\lambda,f}$) qui le représente.

6. Méthode de calibration en ligne d'une interface neuronale directe selon l'une quelconque des revendication précédentes, **caractérisée en ce que** la valeur d'action, $Q_u^{\lambda}$, associée au choix d'une valeur d'hyperparamètre, $\lambda$, à la

$$Q_u^{\lambda} = Q_{u-1}^{\lambda} + \frac{1}{u}\left(R_u^{\lambda} - Q_{u-1}^{\lambda}\right)$$

phase de calibration $u$ est obtenue par $\qquad$, où $Q_{u-1}^{\lambda}$ est la valeur d'action associée à ce choix, lors de la phase de calibration précédente, $R_u^{\lambda}$ est la récompense associée à ce même choix et $u$ est le nombre de phases de calibration effectuées.

7. Méthode de calibration en ligne d'une interface neuronale directe selon l'une quelconque des revendications 1 à 5, **caractérisée en ce que** la valeur d'action, $Q_u^{\lambda}$, associée au choix d'une valeur d'hyperparamètre, $\lambda$, à la phase

$$Q_u^{\lambda} = Q_{u-1}^{\lambda} + \eta\left(R_u^{\lambda} - Q_{u-1}^{\lambda}\right)$$

de calibration $u$ est obtenue par $\qquad$, où $Q_{u-1}^{\lambda}$ est la valeur d'action associée à ce choix, lors de la phase de calibration précédente, $R_u^{\lambda}$ est la récompense associée à ce même choix et $\eta$ est un facteur de mise à jour avec $0 < \eta < 1$.

8. Méthode de calibration en ligne d'une interface neuronale directe selon l'une des revendications précédentes, **caractérisée en ce que** valeur d'hyperparamètre ($\lambda^*$) sélectionnée est obtenue au moyen d'une stratégie gloutonne comme celle correspondant à la valeur d'action la plus élevée sur l'ensemble des valeurs d'hyperparamètre ($\Lambda$).

9. Méthode de calibration en ligne d'une interface neuronale directe selon l'une des revendications 1 à 7, **caractérisée en ce que** la valeur d'hyperparamètre sélectionnée ($\lambda^*$) est obtenue au moyen d'une stratégie $\varepsilon$ - gloutonne, cette valeur étant prise avec une probabilité $\varepsilon$ comme celle correspondant à la valeur d'action la plus élevée sur l'ensemble des valeurs d'hyperparamètre ($\Lambda$) et avec une probabilité ($1$-$\varepsilon$) parmi les autres valeurs, dites sous-optimales.

10. Méthode de calibration en ligne d'une interface neuronale directe selon la revendication 9, **caractérisée en ce qu'**avant l'étape de sélection de la valeur d'hyperparamètre lors de la phase de calibration $u$, chaque valeur d'action,

$$Q_u^\lambda$$

, associée au choix d'une valeur d'hyperparamètre sous-optimale, $\lambda$, est majorée par

$$Q_u'^\lambda = Q_{u-1}^\lambda + \theta . \frac{u - u(\lambda)}{u}$$

où $u(\lambda)$ est l'indice de la dernière phase de calibration dans laquelle la valeur $\lambda$ a été sélectionnée et $\theta$ est un paramètre positif non nul.

**Patentansprüche**

1. Online-Kalibrierungsverfahren für eine direkte neuronale Schnittstelle, die dazu bestimmt ist, eine Vielzahl von elektrophysiologischen Signalen während einer Vielzahl von Beobachtungsfenstern, die mit Beobachtungszeitpunkten verknüpft sind, zu empfangen, um einen Eingabetensor zu bilden und daraus mittels eines Vorhersagemodells, das durch einen Vorhersagetensor dargestellt wird, einen Ausgangstensor abzuleiten, der Steuersignale bereitstellt, die dazu bestimmt sind, einen oder mehrere Effektoren zu steuern, wobei das Kalibrieren während einer Vielzahl von Kalibrierungsphasen ausgeführt wird, wobei jede Kalibrierungsphase **u** anhand von Eingabekalibrierungsdaten,

$$\underline{\mathbf{X}}_u \in \mathbb{R}^{(I_1 \times ... \times I_N)}$$

die durch einen Eingabekalibrierungstensor, , dargestellt werden, und Ausgabekalibrie-

$$\underline{\mathbf{Y}}_u \in \mathbb{R}^{(J_1 \times ... \times J_M)}$$

rungsdaten, die durch einen Ausgabekalibrierungstensor, , dargestellt werden, durchgeführt wird, wobei die Kalibrierungsphase eine bestrafte REW-NPLS-Regression implementiert, wobei eine REW-NPLS-Regression, aus dem Englischen für *"Recursive Exponentially Weighted N-way Partial Least Squares",* ein rekursives, multivariates, exponentiell gewichtetes partielles Regressionsverfahren der kleinsten Quadrate ist, wobei die Bestrafung sich auf mindestens eine Modalität des Vorhersagetensors bezieht und mindestens einen Hyperparameter einbezieht, **dadurch gekennzeichnet, dass** bei einer Kalibrierungsphase Folgendes erfolgt:

$$\Lambda_u^{test}$$

- Extrahieren (110) eine Teilmenge, , von Werten des zu testenden Hyperparameters aus einer Menge, $\Lambda$, von Werten des Hyperparameters;

$$\left( e_u^{\lambda, f} \right)$$

- Berechnen (120) eines Vorhersageleistungsindikators für jedes Vorhersagemodell einer

$$\mathbf{\Theta}_{u-1}^{\lambda, f}$$

Vielzahl von bestraften Vorhersagemodellen, wobei jedes bestrafte Vorhersagemodell ( ) mit einer Dimension (f) des Raums latenter Variablen verknüpft ist und während der vorherigen Kalibrierungsphase, **u** - 1, durch eine bestrafte REW-NPLS-Regression mittels eines Hyperparameterwerts ($\lambda$) der Teilmenge erhalten wurden, wobei der Vorhersageindikator des bestraften Vorhersagemodells die Qualität der Vorhersage des Ausgabekalibrierungstensors durch dieses Modell anhand des Eingabekalibrierungstensors darstellt;

$$R_u^\lambda$$

- Berechnen (130) einer Belohnung ( ), die mit der Auswahl dieses Werts verknüpft ist, anhand der

Vorhersageleistungsindikatoren ( $e_u^{\lambda,f}$ ) der bestraften Vorhersagemodelle ( $\Theta_{u-1}^{\lambda,f}$ ) für jeden Hyperparameterwert ($\lambda$) der Teilmenge;

- für jeden Hyperparameterwert ($\lambda$) der Teilmenge, Aktualisieren (140) eines Aktionswerts ( $Q_u^{\lambda}$ ), der der Auswahl dieses Werts entspricht, anhand der erhaltenen Belohnung ( $R_u^{\lambda}$ ) für die Auswahl dieses Werts im vorherigen Schritt;

- Auswählen (150) gemäß einer Aktionsauswahlstrategie eines Hyperparameterwerts ($\lambda$\*) aus der Menge $\Lambda$ von Hyperparameterwerten anhand der so aktualisierten Aktionswerte ( $Q_u^{\lambda}$ );

- Aktualisieren (160) jedes der bestraften Vorhersagemodelle der Vielzahl mittels einer REW-NPLS-Regression, die mittels eines Hyperparameterwerts ($\lambda$) der Menge $\Lambda$ bestraft wird;

- Verwenden (170) des Vorhersagemodells, das durch den im vorherigen Schritt ausgewählten und aktualisierten Hyperparameterwert bestraft wurde, um einen Steuertensor vorherzusagen, der die Steuersignale anhand des Eingabetensors bereitstellt.

2. Online-Kalibrierungsverfahren für eine direkte neuronale Schnittstelle nach Anspruch 1, **dadurch gekennzeichnet, dass** der Vorhersageleistungsindikator, ( $e_u^{\lambda,f}$ ), eines bestraften Vorhersagemodells, das mit einer Dimension f des Raums latenter Variablen und einem Hyperparameterwert $\lambda$ der Teilmenge verknüpft ist, durch

$$e_u^{\lambda,f} = \mu . e_{u-1}^{\lambda,f} + \delta\left(\underline{\mathbf{Y}}_u, \widehat{\underline{\mathbf{Y}}}_u^{\lambda,f}\right)$$

rekursiv erhalten wird, wobei $\widehat{\underline{\mathbf{Y}}}_u^{\lambda,f}$ die Ausgabetensorvorhersage ist, die anhand des bestraften Vorhersagemodells erhalten wurde, das während der vorherigen Kalibrierungsphase, $\Theta_{u-1}^{\lambda,f}$, erhalten wurde, $\delta\left(\underline{\mathbf{Y}}_u, \widehat{\underline{\mathbf{Y}}}_u^{\lambda,f}\right)$ eine abnehmende Funktion der Lücke zwischen den Tensoren $\underline{\mathbf{Y}}_u$ und $\widehat{\underline{\mathbf{Y}}}_u^{\lambda,f}$ ist und $0 < \mu < 1$ ein Vergessenskoeffizient ist.

3. Online-Kalibrierungsverfahren für eine direkte neuronale Schnittstelle nach Anspruch 1 oder 2, **dadurch gekennzeichnet, dass** die Belohnung ( $R_u^{\lambda}$ ), die mit der Auswahl des Hyperparameterwerts $\lambda$ verknüpft ist, anhand des besten Vorhersageleistungsindikators, $e_u^{\lambda^*,f}$, erhalten wird, der durch die bestraften Vorhersagemodelle $\Theta_{u-1}^{\lambda,f}$ über die Vielzahl der möglichen Dimensionen, f = 1,...,F, des Raums latenter Variablen erhalten wurde.

4. Online-Kalibrierungsverfahren für eine direkte neuronale Schnittstelle nach Anspruch 3, **dadurch gekennzeichnet, dass** die Belohnung $R_u^{\lambda}$ durch $R_u^{\lambda} = \max_{\lambda \in \Lambda}\left(rank\left(e_u^{\lambda}\right)\right) - rank\left(e_u^{\lambda}\right)$ gegeben ist, wobei *rank*(.) eine Sortierfunktion nach absteigenden Werten ist.

5. Online-Kalibrierungsverfahren für eine direkte neuronale Schnittstelle nach Anspruch 1 oder 2, **dadurch gekenn-**

**zeichnet, dass** die Belohnung ($R_u^\lambda$), die mit der Auswahl eines Hyperparameterwerts (λ) verknüpft ist, ferner anhand von Sparsamkeitsindikatoren der bestraften Vorhersagemodelle ($\Theta_{u-1}^{\lambda,f}$) erhalten wird, wobei ein Sparsamkeitsindikator eines bestraften Vorhersagemodells als zunehmende Funktion der Anzahl der Nullelemente des Vorhersagetensors ($\underline{\mathbf{B}}_{u-1}^{\lambda,f}$) definiert ist, der es darstellt.

6. Online-Kalibrierungsverfahren für eine direkte neuronale Schnittstelle nach einem der vorhergehenden Ansprüche, **dadurch gekennzeichnet, dass** der Aktionswert, $Q_u^\lambda$, der mit der Auswahl eines Hyperparameterwerts, λ, in

$$Q_u^\lambda = Q_{u-1}^\lambda + \frac{1}{u}\left(R_u^\lambda - Q_{u-1}^\lambda\right)$$

der Kalibrierungsphase **u** verknüpft ist, durch erhalten wird, wobei $Q_{u-1}^\lambda$ derAktionswert, der mit dieser Auswahl verknüpft ist, während der vorherigen Kalibrierungsphase ist, $R_u^\lambda$ die Belohnung ist, die mit derselben Auswahl verknüpft ist, und **u** die Anzahl der ausgeführten Kalibrierungsphasen ist.

7. Online-Kalibrierungsverfahren für eine direkte neuronale Schnittstelle nach einem der Ansprüche 1 bis 5, **dadurch gekennzeichnet, dass** der Aktionswert, $Q_u^\lambda$, der mit der Auswahl eines Hyperparameterwerts, λ, in der Kalib-

$$Q_u^\lambda = Q_{u-1}^\lambda + \eta\left(R_u^\lambda - Q_{u-1}^\lambda\right)$$

rierungsphase **u** verknüpft ist, durch erhalten wird, wobei $Q_{u-1}^\lambda$ der Aktionswert, der mit dieser Auswahl verknüpft ist, während der vorherigen Kalibrierungsphase ist, $R_u^\lambda$ die Belohnung ist, die mit derselben Auswahl verknüpft ist, und $\eta$ ein Aktualisierungsfaktor ist, bei dem 0 < $\eta$ < 1.

8. Online-Kalibrierungsverfahren für eine direkte neuronale Schnittstelle nach einem der vorhergehenden Ansprüche, **dadurch gekennzeichnet, dass** der ausgewählte Hyperparameterwert (λ*) mittels einer Greedy-Strategie wie derjenigen, die dem höchsten Aktionswert aus der Menge der Hyperparameterwerte (Λ) entspricht, erhalten wird.

9. Online-Kalibrierungsverfahren für eine direkte neuronale Schnittstelle nach einem der Ansprüche 1 bis 7, **dadurch gekennzeichnet, dass** der ausgewählte Hyperparameterwert (λ*) mittels einer ε-Greedy-Strategie erhalten wird, wobei dieser Wert mit einer Wahrscheinlichkeit ε wie diejenige, die dem höchsten Aktionswert aus der Menge der Hyperparameterwerte (Λ) entspricht, und mit einer Wahrscheinlichkeit (1 - ε) unter den anderen Werten, den sogenannten suboptimalen Werten, genommen wird.

10. Online-Kalibrierungsverfahren für eine direkte neuronale Schnittstelle nach Anspruch 9, **dadurch gekennzeichnet, dass** vor dem Schritt des Auswählens des Hyperparameterwerts während der Kalibrierungsphase **u** jeder Aktionswert, $Q_u^\lambda$, der mit der Auswahl eines suboptimalen Hyperparameterwerts, λ, verknüpft ist, um

$$Q'^{\lambda}_{u} = Q^{\lambda}_{u-1} + \theta . \frac{u - u(\lambda)}{u}$$

erhöht wird, wobei $u(\lambda)$ der Index der letzten Kalibrierungsphase ist, in der der Wert $\lambda$ ausgewählt wurde, und $\theta$ ein positiver Parameter ungleich Null ist.

## Claims

1. A method for online calibration of a direct neural interface intended to receive a plurality of electrophysiological signals during a plurality of observation windows associated with observation instants, to form an input tensor and deduce therefrom, by means of a predictive model represented by a prediction tensor, an output tensor providing control signals intended to control one or more effectors, said calibration being carried out during a plurality of calibration phases, each calibration phase u being carried out from input calibration data, represented by an input calibration tensor, $\underline{\mathbf{X}}_u \in \mathbb{R}^{(I_1 \times ... \times I_N)}$ , and output calibration data represented by an output calibration tensor, $\underline{\mathbf{Y}}_u \in \mathbb{R}^{(J_1 \times ... \times J_M)}$ said calibration phase implementing a penalised REW-NPLS regression, a REW-NPLS regression being a Recursive Exponentially Weighted N-way Partial Least Squares method, the penalisation relating to at least one modality of the prediction tensor and involving at least one hyperparameter, **characterised in that**, during a calibration phase:

   - a subset, $\mathbf{\Lambda}^{test}_u$ , of values of the hyperparameter to be tested is extracted (110) from a set, $\Lambda$, of values of the hyperparameter;

   - a prediction performance indicator ( $e^{\lambda,f}_u$ ) is calculated (120) for each predictive model of a plurality of penalised predictive models, each penalised predictive model ( $\mathbf{\Theta}^{\lambda,f}_{u-1}$ ) being associated with a dimension (f) of the space of latent variables and having been obtained, during the previous calibration phase, u-1, by REW-NPLS regression penalised by means of a hyperparameter value ($\lambda$) of said subset, the prediction indicator of the penalised predictive model representing the quality of the prediction of the output calibration tensor by this model from the input calibration tensor;

   - for each hyperparameter value (A) of said subset, a reward ( $R^{\lambda}_u$ ) associated with the choice of this value is calculated (130) from the prediction performance indicators ( $e^{\lambda,f}_u$ ) of the penalised predictive models ( $\mathbf{\Theta}^{\lambda,f}_{u-1}$ );

   - for each hyperparameter value ($\lambda$) of said subset, an action value ( $Q^{\lambda}_u$ ) corresponding to the choice of this value is updated (140) from the reward obtained ( $R^{\lambda}_u$ ) for the choice of this value in the previous step;
   - a hyperparameter value (A*) is selected (150) according to an action selection strategy among the set $\Lambda$ of hyperparameter values from the action values thus updated ( $Q^{\lambda}_u$ );
   - each of the penalised predictive models of said plurality is updated (160), by means of a REW-NPLS regression penalised by means of a hyperparameter value ($\lambda$) of the set $\Lambda$;
   - the predictive model penalised by means of said hyperparameter value selected and updated in the previous step is used (170) to predict a control tensor providing said control signals from said input tensor.

2. The method for online calibration of a direct neural interface according to claim 1, **characterised in that** the prediction performance indicator, $e^{\lambda,f}_u$ , of a penalised predictive model associated with a dimension f of the space of latent variables and with a hyperparameter value A of the subset is obtained recursively by

$$e_u^{\lambda,f} = \mu \cdot e_{u-1}^{\lambda,f} + \delta\left(\underline{\mathbf{Y}}_u, \underline{\widehat{\mathbf{Y}}}_u^{\lambda,f}\right)$$ where $\underline{\widehat{\mathbf{Y}}}_u^{\lambda,f}$ is the output tensor prediction obtained from the penalised predictive model obtained during the previous calibration phase, $\Theta_{u-1}^{\lambda,f}$, $\delta\left(\underline{\mathbf{Y}}_u, \widehat{\mathbf{Y}}_u^{\lambda,f}\right)$ is a decreasing function of the difference between the tensors $\underline{\mathbf{Y}}_u$ and $\widehat{\mathbf{Y}}_u^{\lambda,f}$, and $0 < \mu < 1$ is a forgetting coefficient.

3. The method for online calibration of a direct neural interface according to claim 1 or 2, **characterised in that** the reward $R_u^{\lambda}$ associated with the choice of the hyperparameter value $\lambda$, is obtained from the best prediction performance indicator, $e_u^{\lambda^*,f}$, obtained by the penalised predictive models $\Theta_{u-1}^{\lambda,f}$, on the plurality of possible dimensions, $f = 1, \dots, F$ of the space of latent variables.

4. The method for online calibration of a direct neural interface according to claim 3, **characterised in that** the reward $R_u^{\lambda}$ is given by $$R_u^{\lambda} = \max_{\lambda \in \Lambda}\left(\mathrm{rank}\left(e_u^{\lambda}\right)\right) - \mathrm{rank}\left(e_u^{\lambda}\right)$$ where rank (.) is a function for sorting by decreasing values.

5. The method for online calibration of a direct neural interface according to claim 1 or 2, **characterised in that** the reward ($R_u^{\lambda}$) associated with the choice of a hyperparameter value (A) is further obtained from parsimony indicators of the penalised predictive models $\Theta_{u-1}^{\lambda,f}$, a parsimony indicator of a penalised predictive model being defined as an increasing function of the number of zero elements of the prediction tensor $\underline{\mathrm{B}}_{u-1}^{\lambda,f}$ which represents it.

6. The method for online calibration of a direct neural interface according to any one of the preceding claims, **characterised in that** the action value, $Q_u^{\lambda}$, associated with the choice of a hyperparameter value, $\lambda$, at the phase of calibration u is obtained by $$Q_u^{\lambda} = Q_{u-1}^{\lambda} + \frac{1}{u}\left(R_u^{\lambda} - Q_{u-1}^{\lambda}\right)$$, where $Q_{u-1}^{\lambda}$ is the action value associated with this choice, during the previous calibration phase, $R_u^{\lambda}$ is the reward associated with this same choice and u is the number of calibration phases carried out.

7. The method for online calibration of a direct neural interface according to any one of claims 1 to 5, **characterised in that** the action value, $Q_u^{\lambda}$, associated with the choice of a hyperparameter value, A, at the calibration phase u is obtained by $$Q_u^{\lambda} = Q_{u-1}^{\lambda} + \eta\left(R_u^{\lambda} - Q_{u-1}^{\lambda}\right)$$, where $Q_{u-1}^{\lambda}$ is the action value associated with this choice, during the previous calibration phase, $R_u^{\lambda}$ is the reward associated with this same choice and $\eta$ is an update factor with $0 < \eta < 1$.

8. The method for online calibration of a direct neural interface according to one of the preceding claims, **characterised in that** the hyperparameter value ($\lambda^*$) selected is obtained by means of a greedy strategy such as that corresponding to the highest action value on the set of hyperparameter values ($\Lambda$).

9. The method for online calibration of a direct neural interface according to one of claims 1 to 7, **characterised in that** the selected hyperparameter value ($\lambda^*$) is obtained by means of an $\varepsilon$-greedy strategy, this value being taken

with a probability $\varepsilon$ as that corresponding to the highest action value on the set of hyperparameter values ($\Lambda$) and with a probability ($1-\varepsilon$) among the other values, called suboptimal values.

10. The method for online calibration of a direct neural interface according to claim 9, **characterised in that** before the step of selecting the hyperparameter value during the calibration phase u, each action value, $Q_u^\lambda$, associated with the choice of a suboptimal hyperparameter value, $\lambda$, is increased by

$$Q_u'^\lambda = Q_{u-1}^\lambda + \theta \cdot \frac{u-u(\lambda)}{u}$$

where $u(\lambda)$ is the index of the last calibration phase in which the value $\lambda$ was selected and $\theta$ is a non-zero positive parameter.

**Figure unique**

**EP 4 111 976 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- WO 2013057544 A
- FR 2010497

### Littérature non-brevet citée dans la description

- **P. GELADI et al.** Partial least-squares régression : a tutorial. *dans Analytica Chimica Acta,* 1986, vol. 185, 1-17
- **DE S. VANCOLEN.** *La régression PLS,* Juin 2004
- **R. BRO.** *Multi-way analysis in the food industry : models, algorithms and applications,* 1998
- **DE A. ELISEYEV ; AKSENOVA.** Recursive N-Way Partial Least Squares for Brain-Computer Interface. *dans PLOS One,* Juillet 2013, vol. 8 (7), e69962
- **DE A. ELISEYEV et al.** Recursive exponentially weighted N-way partial least squares régression with recursive hyperparameters in Brain- Computer Interface applications. *Sci. Rep,* Novembre 2017, vol. 7 (1), 16281
- **DE A. MOLY et al.** On-line adaptive group-wise sparse NPLS for ECoG neural signal decoding. *arXiv.org,* 14 Octobre 2020
- Online adaptive group-wise sparse NPLS for ECoG neural signal decoding. **D'ALEXANDRE MOLY et al.** ARXIV.ORG. CORNELL UNIVERSITY LIBRARY, 14 Octobre 2020